Europäisches Patentamt

European Patent Office

Office européen des brevets

(19)

(11) Numéro de publication: **0 070 245**
**A1**

## (12) DEMANDE DE BREVET EUROPEEN

(21) Numéro de dépôt: **82420084.4**

(22) Date de dépôt: **28.06.82**

(51) Int. Cl.³: **C 12 M 1/00**

(30) Priorité: **02.07.81 FR 8113293**
**12.02.82 FR 8202464**

(43) Date de publication de la demande:
**19.01.83 Bulletin 83/3**

(84) Etats contractants désignés:
**IT**

(71) Demandeur: **Guerin, Maurice**
**53 rue Victor Hugo**
**F-71000 Macon(FR)**

(71) Demandeur: **Ribaud, Patricia Catherine**
**20 rue Emile Dubois**
**F-75014 Paris(FR)**

(72) Inventeur: **Guerin, Maurice**
**53 rue Victor Hugo**
**F-71000 Macon(FR)**

(72) Inventeur: **Ribaud, Patricia Catherine**
**20 rue Emile Dubois**
**F-75014 Paris(FR)**

(54) **Digesteur continu pour la production de biométhane à partir de substance organique.**

(57) - Il est composé d'une cuve parallélipipédique cloisonnée à grand axe horizontal; les produits passent successivement d'un compartiment à l'autre. Le compartiment où s'effectue principalement la digestion est séparé en 2 parties 27 et 28 par la paroi mobile 32. Une pompe permet d'envoyer les produits en voie de fermentation successivement de part et d'autre de cette paroi en même temps que sont introduits les produits à digérer dans la veine des produits en cours de recirculation.

EP 0 070 245 A1

./...

Croydon Printing Company Ltd

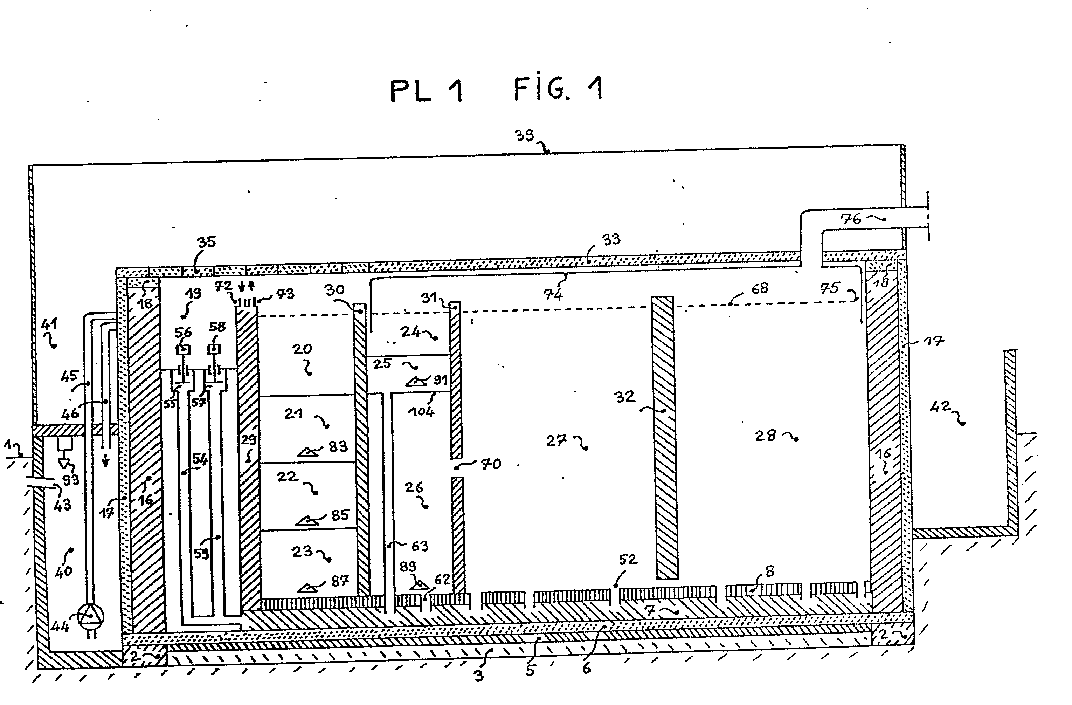

PL 1   FIG. 1

- 1 -

## TITRE DE L'INVENTION

DIGESTEUR CONTINU POUR LA PRODUCTION DE BIOMETHANE A PARTIR DE SUBSTANCES ORGANIQUES.

### DOMAINES TECHNIQUES AUXQUELS L'INVENTION SE RAPPORTE :

La présente invention concerne les appareils destinés à produire en continu du biométhane à partir de substances organiques contenues dans des déchets animaux ou végétaux provenant essentiellement de l'agriculture ou des industries dérivées de celle-ci.

Elle concerne également les systèmes d'épuration des effluents fortement chargés de matières organiques puisque la fermentation méthanogène s'accompagne d'une forte réduction de la $DBO_5$.

### ETAT DE LA TECHNIQUE

Actuellement les digesteurs continus sont de 2 types :

- Les digesteurs verticaux à chambre unique ou à double chambre.

- Les digesteurs horizontaux ou encore appelés " à déplacement".

-Actuellement les appareils continus existants ont en commun au moins une ou plusieurs des difficultés suivantes :

- Difficultés avec la croûte : La destruction de la croûte qui se forme à la surface des produits en cours de digestion est parfois réalisée de façon insuffisante ; il en résulte des troubles de fonctionnement.

- Difficultés avec les boues du fond : Parfois ces boues sont éliminées de façon insuffisante, elles sédimentent, durcissent et le volume utile de digestion diminue peu à peu.

-Bilan thermique : Le chauffage des produits entrant à la température de digestion représente une dépense énergétique importante ; il est donc intéressant de récupérer de la chaleur sur les produits sortants ; c'est un point qui a été généralement négligé jusqu'à présent.

-Absence de maîtrise de l'hydraulique interne .

Généralement on ne sait pas comment se répartissent les entrées dans le volume des produits présents dans le digesteur, ni qu'elle est l'efficacité des différents systèmes de brassage utili- sé. Au point de vue de la biologie l'objectif à atteindre est cependant des entrées réparties par petite quantité de façon homogène dans tout le volume du digesteur.

MOYENS MIS EN OEUVRE

Ce digesteur ne traite que des produits manipulables par pompe.

Les produits à digérer arrivent dans la fosse de régulation 40 par la canalisation 43. Le niveau est mesuré en permanence par le contrôleur de niveau 93. Le rôle de la fosse de régulation est de permettre de répartir le mieux possible pendant une période déterminée les entrées de produits frais dans le digesteur. Les produits frais sont repris par la pompe 44 qui par l'intermédiaire de la canalisation 45 alimente à refus le canal d'entrée 48 de l'échangeur de chaleur. Le niveau est maintenu constant par une lame déversante 47, les produits en excès retournent à la fosse de régulation 40 par la canalisation 46. Les produits frais circulent tout au long de ce canal d'entrée 48 où ils s'échauffent en prélevant des calories sur les produits sortants qui circulent à contre courant dans le canal de sortie 65 de l'échangeur. Afin de faciliter les échanges thermiques les produits dans les canaux 48 et 65 sont brassés par les palettes 78 et 79 qui font en position de brassage un angle de 45° par rapport au plan médian longitudinal de chaque canal. Pour faciliter l'avancement des produits dans ces mêmes canaux périodiquement ces mêmes palettes 78 et 79 successivement dans chaque canal poussent les produits devant elles, pour cela elles avancent en faisant un angle de 90° par rapport au plan médian longitudinal des canaux.

Les produits pénètrent ensuite dans l'étage supérieur 21 du compartiment primaire. Les produits y sont brassés par la pale 83. Les produits parcourent ensuite l'étage moyen 22 du compartiment primaire, ils y sont brassés par la pale 85, puis l'étage inférieur 23 de ce même compartiment où les produits sont brassés par la pale 87. Dans ce compartiment primaire où les produits séjournent environ 36 heures les produits frais subissent des réactions de dépolymérisation et d'acidification. Les produits entrants à l'étage supérieur 21 sont ensemencés avec environ 10 % de produits prélevés à l'étage inférieur 23. Dans ce compartiment primaire les produits achèvent de se mettre à la température de digestion, la chaleur étant fournie par le plancher chauffant qui se situe à la base de l'étage inférieur 23.

La digestion méthanogène se réalise dans les compartiments secondaire et tertiaire. Le compartiment secondaire présente la caractéristique suivante : il est divisé en 2 volumes variables 27 et 28 par une paroi mobile 32 qui se déplace alternativement d'avant en arrière puis d'arrière en avant en balayant à chaque fois la totalité du volume du compartiment secondaire moins à chaque extrêmité un volume résiduel qui se trouve enfermé entre la paroi fixe et la paroi mobile. Au fond du compartiment secondaire débouche dans l'axe des canalisations qui permettent d'aspirer les produits devant la paroi mobile et de les refouler derrière la paroi mobile. La même canalisation sert successivement à l'aspiration puis au refoulement. Toutes les canalisations peuvent être, par un ensemble de vannes reliées à la pompe de recirculation 67. Le volume balayé par la paroi mobile pendant un temps donné est égal au débit de la pompe 67 pendant ce même temps ; ce qui fait que, lorsque la paroi mobile s'est déplacée d'une extrêmité à l'autre, l'ensemble du volume du compartiment secondaire est passé dans la pompe 67 de recirculation pour être envoyé à nouveau dans ce même compartiment. Les fractions supérieures des volumes résiduels dont il a été question plus haut sont prélevés par les canalisations 61 et envoyées dans le compartiment tertiaire de même les fractions inférieures sont prélevées par les canalisations situées au fond et aux extrêmités et envoyées dans le compartiment tertiaire. Au cours de chaque transfert des produits du compartiment secondaire de part et d'autre de la paroi mobile une pompe volumétrique 50 injecte dans l'aspiration de la pompe de recirculation 67 les produits qui sont prélevés à la sortie de l'étage inférieur du compartiment primaire. Le rapport entre les débits de la pompe d'injection et la pompe de recirculation dépend du nombre d'aller et retour que l'on fait faire à la paroi mobile pendant un temps déterminé. De toute façon pour avoir un fonctionnement optimum sur le plan biologique on a intérêt à ce que ce rapport soit faible c'est-à-dire inférieur à 5 % de façon à ne pas déborder le pouvoir tampon des produits en cours de digestion et donc éviter l'abaissement du Ph dû à l'introduction des produits en provenance du compartiment primaire. Rappelons que l'abaissement du Ph en-dessous de la neutralité est défavorable à l'activité des bactéries méthanogènes.

Dans le compartiment tertiaire 26, les produits achèvent leur fermentation méthanogène sans apport de produits en provenance du compartiment primaire. Ils sont brassés par la pale 89.

Dans le compartiment tertiaire les fractions légères gagnent la partie supérieure 24 de ce compartiment. A la surface de ce compartiment tertiaire un dispositif empêche la formation d'une croûte et permet le passage des fractions les plus légères sous le rebord de la bâche 74 de récupération du gaz. car pour assurer la formation d'un joint hydraulique le bord de la bâche plonge dans le liquide. Ce dispositif constitué par une plaque 115 et par une goulotte 118 fixée sur celle-ci racle les flottants à la surface en se déplaçant horizontalement de la position 118 A à la position 118 B où elle s'enfonce en se rapprochant de la paroi 30. Au cours de ce mouvement la goulotte 118 emprisonne les flottants et par un mouvement de bascule en 118 C les libère dans le canal de sortie 65 de l'échangeur de chaleur. La plaque revient ensuite par un parcours horizontal dans le liquide à son point de départ et le cycle recommence périodiquement.

Les fractions lourdes constituées par les boues se déposent au fond du compartiment tertiaire. Elles sont reprises par la pompe de recirculation 67 grâce à la canalisation 62 et envoyées avec la canalisation 63 dans le bac 25 d'alimentation du canal de sortie de l'échangeur. Dans ce bac la pale de brassage 91 les empêche de sédimenter et leur permet de s'évacuer dans le canal de sortie de l'échangeur de chaleur.

Les produits en parcourant le canal de sortie de l'échangeur cèdent une partie de leur chaleur aux produits entrants. Le niveau est maintenu constant dans ce canal par la lame déversante 66 qui est située à sa sortie. Par construction et du fait des communications : échancrure 64 dans la cloison 30 : trous 70 dans la cloison 31 ; passage 69 dans la partie supérieure de la paroi mobile 32 , ce niveau est le niveau commun 68 à l'ensemble des produits dans le digesteur. Il est aussi celui du canal d'entrée 48 de l'échangeur de chaleur puisque les lames déversantes 66 et 47 sont au même niveau.

Les produits sont maintenus à la température de digestion par un plancher chauffant qui occupe tout le fond des compartiments primaire, secondaire et tertiaire.

Tous les organes de brassages : palettes 78 et 79 des canaux d'entrée et de sortie de l'échangeur de chaleur, les pales de brassages 83, 85, 87 des étages du compartiment primaire, la pale 89 du compartiment tertiaire, la pale 91 du bac d'alimentation 25, la plaque 115 sont solidaires mécaniquement du même bâti mobile qui est animé de mouvements rectilignes alternatifs lents.

Le gaz produit par la digestion est recueilli à la surface des compartiments secondaires et tertiaires par une bâche souple 74 dont les bords plongent dans le liquide assurant ainsi l'étanchéïté par un joint hydraulique. Le gaz est conduit par la conduite 76 dans une enceinte 125 à la température ambiante où la vapeur d'eau se condense en partie ; l'eau de condensation s'élimine automatiquement par un siphon 126. Le gaz est ensuite stocké dans un réservoir souple 127. Il est ensuite au fur et à mesure des besoins épuré et mis en pression par un système qui réalise en même temps l'épuration par barbotage dans de l'eau et une légère mise en pression (de l'ordre de 100 millibars) suffisante pour une utilisation facile sur place.

Ce système est composé d'un certain nombre d'augets 137 qui décrivent un mouvement circulaire. Alternativement ces augets se remplissent de gaz : position 137 A puis ils s'enfoncent dans l'eau. La pression hydro statique assure la compression du gaz. Au point le plus bas (position 137 C) l'auget bascule et libère le gaz, celui-ci est recueilli à la base de la cloche de récupération 142. Le gaz en s'élevant traverse un diffuseur 143 qui divise le flux de gaz en petites bulles ; ces petites bulles montent dans le liquide, leur parcours est allongé par la présence de chicanes 144 situées dans l'eau. Au cours de ce parcours le biogaz cède à l'eau la majeure partie des gaz solubles que l'on désire éliminer, c'est-à-dire. $SH_2$ et $CO_2$.

L'épuration se complète par contact avec l'eau qui ruisselle sur les chicanes 145 situées en atmosphère gazeuze. Le gaz épuré sous pression est recueilli par la cloche 149 , la pression qui s'exerce sur

- 6 -

le gaz est fonction des différences de niveau entre les niveaux 151 et 152. Le gaz est ensuite envoyé dans un gazomètre constitué par une cloche lestée et qui se déplace verticalement dans le même bac 42. Puis il part vers les utilisations. L'eau contenant les gaz dissous est éliminée par la conduite 146 . Elle est remplacée par de l'eau neuve ou régénérée amenée par la canalisation 147.

L'accessibilité à l'intérieur est obtenue par des panneaux basculants 38 qui constituent la couverture puis par des panneaux isolants amovibles 34 qui permettent la visite des parties supérieures latérales et des panneaux isolants amovibles 35 qui permettent l'accès au compartiment des vannes et à l'échangeur de chaleur.

AVANTAGES APPORTES PAR L'INVENTION

La particularité fondamentale est l'utilisation d'une paroi mobile avec déplacement régulier de la quasi totalité de la masse des produits en digestion alternativement de part et d'autre de cette paroi à l'aide d'une pompe et d'un ensemble de canalisations fixes ; ceci a pour conséquences principales : la disparition totale du problème de la croûte puisque la croûte est intégralement détruite mécaniquement lors des déplacements de la paroi mobile et que ces débrits sont aspirés à chaque extrémité pour être ensuite rejetée assez rapidement à l'extérieur ; la disparition du problème posé par la sédimentation des boues au fond du digesteur, sédimentation qui aboutit à la diminution du volume utile de digestion ; ce problème est résolu de la même façon que le problème de la croûte ; mécaniquement les boues ne peuvent plus sédimenter.

Ce qui est fondamental, c'est que l'on maitrise l'hydraulique interne du digesteur et que l'on place les bactéries méthanogènes dans des conditions optimum de rendement par un apport régulier et homogène de nutriments dans toute la masse en digestion. Ces nutriments sont débarassés de leur oxygène dissout, ce qui est très important car les bactéries méthanogènes sont sensibles à la moindre trace d'oxygène (Rappelons que cet oxygène a été absorbé par les bactéries formatrices d'acides dans le compartiment primaire et que dans ce compartiment primaire se développeront des populations spécifiques). Les apports étant dilués de façon homogène dans tout le volume de digestion, et dilués à faible dose on a ainsi une utilisation optimum du pouvoir tampon de la masse des produits en digestion ; il sera donc facile de maintenir le Ph des produits en cours de digestion dans des zones de Ph favorable aux bactéries méthanogènes, on évitera ainsi la formation de poches acides dans lesquelles et au voisinage desquelles la digestion méthanogène est fortement ralentie. En définitive on évite la formation de zones mortes dans le digesteur ce qui est bien sûr un facteur très favorable au rendement.

Le digesteur constitue un ensemble parallélipipédique, très compact facile à construire, à isoler et possédant une très bonne accessibilité.

L'intégration aux structures du digesteur d'un dispositif de récupération de la chaleur sur les produits sortants est à même d'améliorer le rendement thermique globale de ces appareils.

Le système d'épuration mise en pression du gaz présente de l'intérêt car ce système est simple et robuste. Il permet d'avoir la pression minimum au-dessus des produits en fermentation, ceci est favorable à la production du gaz et n'impose pas des efforts mécaniques importants sur les structures supérieures du digesteur. Les forces mises en jeu devenant vite importantes dès que la presssion augmente tant soit peu. Ce système remplace les surpresseurs présents à l'heure actuelle sur le marché qui sont coûteux, sujets à corrosion et mal adaptés aux faibles pressions et aux faibles débits.

ENONCE DES FIGURES :

- La figure 1 planche 1 est une coupe verticale longitudinale.
- La figure 2 planche 2 est une coupe horizontale schématique.

Le plan de coupe se confond avec le plan déterminé par la surface des produits en cours de fermentation.

- La figure 3 planche 3 est une demi-coupe verticale passant par le compartiment secondaire.
- La figure 4 planche 3 est une demi-coupe verticale passant par l'échangeur et les 3 étages du compartiment primaire.
- La figure 5 planche 4 est une coupe verticale partielle passant par l'axe vertical des galets porteurs.
- La figure 6 planche 4 est le schéma du circuit du gaz depuis la sortie du digesteur jusqu'à l'utilisation.
- La figure 7 planche 5 est une coupe verticale schématique à travers le système épuration compression, le plan de coupe passe par les godets et est perpendiculaire aux axes supportant ceux-ci.

- La figure 8 planche 5 est une coupe verticale schématique à travers le système épuration compression. Le plan de coupe passe les axes supportant 2 godets symétriques.

- La figure 9 planche 6 est une vue de dessus schématique du chariot porteur des organes de brassage.

- La figure 10 planche 6 est une coupe verticale partielle passant par l'axe horizontal d'un galet porteur du chariot précédent.

- La figure 11 planche 7 est une coupe schématique partielle du sommet du compartiment tertiaire. Le plan de coupe est vertical, parallèle à l'axe longitudinale du digesteur et passe par l'échancrure pratiquée à travers la paroi séparant le compartiment tertiaire de l'échangeur de chaleur pour permettre le passage des produits. Elle a pour but de représenter le brise croûte.

- La figure 12 planche 7 est une vue en perspective de la goulotte 118 qui est fixée à la plaque rectangulaire qui rassemble les flottants à la surface du compartiment tertiaire.

- La figure 13 planche 7 est une coupe partielle horizontale schématique passant par la plaque citée ci-dessus.

- La figure 14 planche 8 est une coupe verticale à travers l'échangeur de chaleur passant par le plan médian du compartiment primaire.

- La figure 15 planche 8 est une coupe verticale passant par le plan médian du canal de sortie de l'échangeur, c'est l'extrémité du canal près de la lame déversante qui est représentée.

- La figure 16 planche 8 représente une vue schématique des palettes qui assurent le brassage et l'avancement des produits dans les canaux d'entrée et de sortie de l'échangeur de chaleur.

- La figure 17 planche 8 représente schématiquement les palettes en position de brassage dans les canaux d'entrée et de sortie de l'échangeur.

- La figure 18 planche 8 représente schématiquement la position des palettes lors de l'avancement des produits dans le canal d'entrée 48.

- La figure 19 planche 8 représente schématiquement la position des palettes lors de l'avancement des produits dans le canal de sortie 65.

DESCRIPTION DETAILLEE

================================================

DESCRIPTION DETAILLEE

Sur la figure 1 planche 1

    - On a représenté le niveau du sol en 1 l'appareil étant conçu pour être mi-enterré. Il est constitué principalement par une cuve parallélipipédique dont l'axe principal est horizontal. A l'extrêmité de la cuve la plus proche du compartiment 19 ; et accolée à la face transversale externe 16 se trouve la fosse de régulation 40 surmontée du local technique 41. Dans la fosse de régulation 40 on a : la canalisation 43 d'amenée de l'effluent ; la pompe de relevage 44 par l'intermédiaire de la canalisation 45 alimente à refus le canal d'entrée de l'échangeur de chaleur, les retours depuis la lame déversante située à l'entrée du canal s'effectuant par la canalisation 46. A l'autre extrêmité est accolée la cuve à eau 42 qui reçoit le système d'épuration mise en pression du biogaz ainsi que le ou les gazomètres tampons.

    - Les parois latérales du digesteur prennent appui sur le sol par l'intermédiaire d'une couronne rectangulaire en béton 2. La section verticale de cette couronne est rectangulaire ; cette section a comme dimensions horizontales la largeur des parois latérales à la base augmentée de l'épaisseur de l'isolant. Le volume situé à l'intérieur de cette couronne est occupé à la partie inférieure par un drainage 3, le reste de la hauteur est occupé par une dalle 5 en béton. Toute la surface de la couronne 2 et de la dalle 5 est recouverte par une couche continue d'isolant incompressible 6. Les parois latérales externes en béton (parois transversales 16 et parois longitudinales) prennent appui à leur base sur la périphérie de la couche isolante 6 et le reste de la couche isolante 6 est surmonté par une dalle 7 en béton dans laquelle sont noyés tous les tubes de transfert de produits. La dalle 7 en béton est surmontée par une dalle 8 en béton dans laquelle sont noyés les tubes de chauffage des produits en cours de digestion sauf sur la surface occupée par le compartiment 19.

- Les parois latérales externes 16 en béton sont revêtus à leur partie extérieure d'une couche continue d'isolant incompressible 17 et à leur partie supérieure d'une couche continue 18 du même isolant.

- Le cloisonnement sur toute la section est perpendiculaire à l'axe longitudinal de la cuve et est réalisé avec successivement les parois fixes 29, 30 et 31 et la paroi mobile 32. De gauche à droite on rencontre successivement et dans l'ordre : un compartiment 19 où sont logées principalement les vannes puis un compartiment qui est occupé à l'étage supérieur par l'échangeur de chaleur 20 et en-dessous par le compartiment appelé compartiment primaire (volumes 21, 22 et 23), puis un compartiment appelé compartiment tertiaire 26 et enfin un compartiment appelé compartiment secondaire divisé en deux (volumes variables 27 et 28). Les produits en cours de digestion passent successivement : dans l'échangeur de chaleur 20, dans le compartiment primaire (volumes 21, 22, 23), dans le compartiment secondaire, dans le compartiment tertiaire (volume 26) puis de nouveau dans l'échangeur 20 où ils cèdent de la chaleur aux produits entrants.

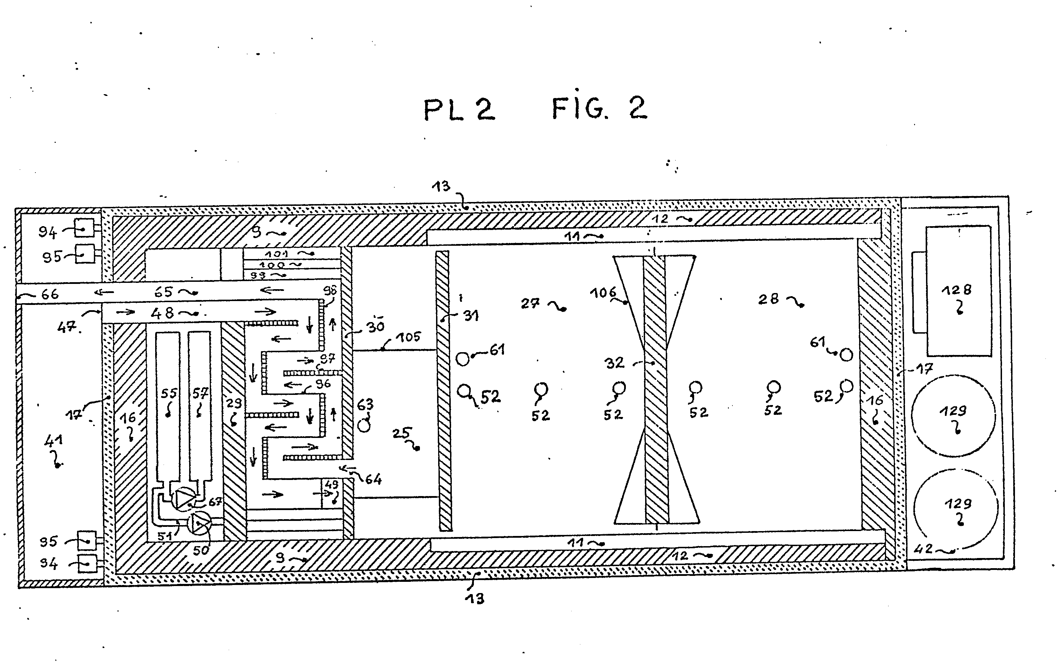

- Le compartiment primaire situé sous l'échangeur de chaleur 20 a une hauteur égale au trois-quart de la hauteur déterminée par le niveau constant 68 du liquide ; il est divisé en 3 étages de même hauteur intérieure par 2 cloisons horizontales qui déterminent donc : un étage supérieur 21, un étage moyen 22 et un étage inférieur 23 ; son volume total est tel que les produits y séjournent environ 36 heures. Les produits passent de l'étage supérieur 21 à l'étage moyen 22 puis à l'étage inférieur 23 par des ouvertures situées de telle façon que le parcours des produits soit maximum. Ils sont prélevés à l'étage inférieur par une canalisation reliée à une pompe volumétrique 50 (figure 2) qui les envoie à l'aide de 2 canalisations équipées chacune d'une vanne: pour 10% du volume à l'entrée de l'étage supérieur du compartiment primaire 21 pour ensemencer les produits frais; pour 90 % du volume dans la canalisation reliée à l'aspiration de la pompe de recirculation 67 (figure 2) avec la canalisation 51 (figure 2)

.- Chacun des 3 étages 21, 22 et 23 est brassé par des pales 83, 85, 87 animées d'un mouvement rectiligne alternatif ; les bras de fixation des pales qui les soutiennent et les entrainent mécaniquement passent dans les espaces libres 99, 100 et 101 représentés sur la figure 2

- Le compartiment secondaire est divisé en 2 sous-compartiments 27 et 28 de volume variable par une cloison mobile 32 qui peut se déplacer d'un mouvement rectiligne alternativement d'un bout à l'autre de ce compartiment jusqu'à 20 cm environ de chaque extrêmité. Le déplacement se fait parallèlement à l'axe longitudinal du digesteur. Du fond du compartiment secondaire en 52 partent des canalisations qui après un parcours horizontal dans la dalle 7 située immédiatement au-dessus de l'isolant 6 arrivent toutes à la base du compartiment 19 ; là, elles se divisent chacune en 2 tronçons ascendants 54 et 59, l'un 54 par l'intermédiaire de vannes 56 est relié au collecteur général d'aspiration 55, l'autre 59 par l'intermédiaire de vannes 58 est relié au distributeur général de refoulement 57. En outre à chaque extrêmité du compartiment secondaire part une canalisation ; chacune a un départ qui se situe à 20 cm en-dessous du niveau général 68 des produits puis elle descend verticalement à peu près dans le plan médian près de la paroi, effectue son parcours horizontal dans la dalle 7 et remonte ensuite verticalement dans le compartiment 19 où par l'intermédiaire de vannes elles sont reliées au collecteur général d'aspiration 55 la pompe de recirculation 67 (voir figure 2) aspire les produits devant la cloison mobile 32 pour les refouler derrière en même temps que sont injectés dans la veine des produits en recirculation, les produits en provenance du compartiment primaire à l'aide de la pompe d'introduction 50 (voir figure 2)

- Le volume du compartiment tertiaire 26 est tel que les produits y séjournent 1 jour ou 2 si le but recherché est la production de biométhane, et qu'en épuration son volume est fonction de la diminution de DBO5 recherché. Du fond du compartiment part une canalisation 62 qui a exactement les mêmes caractéristiques de parcours et de relations que les canalisations qui partent du fond du compartiment secondaire.

- Le niveau général des produits dans l'ensemble du digesteur est représenté en 68 et on voit à travers la paroi 31 le trou de communication 70 de 20 cm de diamètre environ qui permet l'équilibrage des niveaux de part et d'autre de la paroi 31. (Il est situé au centre de la paroi).

- Le fond 104 du bac d'alimentation est sensiblement à la même hauteur que le fond du canal de sortie de l'échangeur de chaleur, il est constitué de 2 panneaux légèrement inclinés l'un vers l'autre de façon à faire à leur ligne de jonction une petite rigole elle-même inclinée vers l'entrée du canal de sortie de l'échangeur de chaleur 20. Les parois latérales verticales 105 de ce bac ont des dimensions telles que leurs faces supérieures horizontales soient à environ la moitié de la hauteur du canal de sortie de l'échangeur de chaleur. Une canalisation 63 alimente par le fond le bac d'alimentation 25. Cette canalisation est reliée au distributeur général de refoulement 57 par l'intermédiaire d'une vanne. Il est également alimenté par surverse au-dessus de la face supérieure horizontale de ses parois latérales, les dites faces supérieures se trouvant en-dessous du niveau général 68 des produits.

- Le fond du compartiment tertiaire 26 et le bac d'alimentation 25 du canal de sortie de l'échangeur sont brassés respectivement par les pales 89 et 91 animées d'un mouvement rectiligne alternatif.

- Toutes les surfaces libres des liquides dans l'ensemble du digesteur sont au même niveau général 68.

- En résumé toutes les canalisations d'aspiration des compartiments secondaire et tertiaire sont reliées au collecteur général d'aspiration 55, à chaque jonction ou à une vanne. Toutes les canalisations de refoulement des compartiments secondaire et tertiaire et du bac 25 sont reliées au distributeur général de refoulement 57, à chaque jonction on a une vanne.

- Le collecteur général d'aspiration 55 et le distributeur général de refoulement 57 sont situés côte à côte dans le quart supérieur du compartiment 19. Ce sont 2 canalisations à section rectangulaire. Leur axe longitudinal est perpendiculaire à l'axe longitudinal du digesteur. Elles sont en charge de 50 cm environ. Toutes les canalisations qui s'y raccordent débouchent à travers leur paroi horizontale inférieure. A chaque débouché de canalisation on a une vanne dont l'élément obturateur est constitué par un disque animé de mouvements rectilignes alternatifs.

- La récupération du gaz se fait au-dessus des compartiments secondaire et tertiaire avec la bâche de récupération 74 dont les bords latéraux plongent dans les produits assurant l'étanchéité grâce à un joint hydraulique 75, le biogaz est évacué grâce à la canalisation 76 dont le départ se situe à travers la face supérieure de la bâche.

- Le départ des tubes du circuit de chauffage est représenté en 72 tandis que l'arrivée de ces mêmes tubes est représenté en 73 à la partie supérieure de la paroi 29.

-Au-dessus du compartiment 19 et au-dessus de l'échangeur de chaleur 20 l'isolation est réalisée par des panneaux isolants amovibles 35 qui laissent un volume libre de 0,50 m environ au-dessus du niveau général des produits. La partie supérieure au-dessus de la bâche 74 de récupération du gaz est isolée par des panneaux isolants fixes 33 ; le volume laissé libre au-dessus du niveau général des produits est de 0,30 m environ.

Figure 2 planche 2

- Sur cette coupe horizontale le plan de coupe est confondu avec le plan déterminé par la surface libre des produits en cours de digestion.

- Les parois longitudinales externes en face du compartiment 19, du compartiment primaire (volumes 21, 22, 23), du compartiment tertiaire 26, sont constituées chacune jusqu'à une hauteur supérieure de quelques centimètres au niveau général 68 des

- 14 -

produits par une paroi 9 en béton au-dessus le reste est constitué par une paroi en béton située à l'extérieur et d'épaisseur
égale à la moitié de celle de la paroi 9. Le décrochement dont
la largeur est égale à la moitié de l'épaisseur de la paroi 9
est situé à l'intérieur.

- Les parois longitudinales externes situées en face du
compartiment secondaire sont constituées jusqu'à une hauteur inférieure de 50 cm environ au niveau général 68 des produits par
une paroi en béton 11 de même épaisseur que la paroi 9 citée
plus haut, au-dessus le reste est constitué par une paroi 12 en
béton située à l'extérieur et d'épaisseur égale à la moitié de la
paroi 11. Le décrochement dont la largeur est égale à la moitié
de l'épaisseur de la paroi 11 est situé vers l'intérieur.

- Toute la surface externe des parois latérales en béton de la cuve est recouverte de couches continues 13 et 17
d'isolant incompressible.

- L'échangeur de chaleur occupe un volume situé au-dessus du compartiment primaire, ce volume a pour surface de base la
surface du compartiment primaire moins une bande de 30 cm de large
contre chacune des faces latérales et pour hauteur le quart de la
hauteur déterminé par le niveau constant 68 du liquide (voir
fig. 1).

- Au début du canal d'entrée 48 on a une lame déversante 47 dont la face supérieure horizontale est exactement au
même niveau que le niveau général 68 des liquides dans l'ensemble du digesteur. Ce canal de section rectangulaire constante a
pour largeur 10 cm environ et pour hauteur la hauteur de l'échangeur. A son début ce canal traverse le compartiment 19 ; il pénêtre ensuite dans la zone occupée par l'échangeur proprement dit
qu'il parcourt en faisant des allers et retours successifs de façon à parcourir la distance maximum, les produits le parcourt dans
le sens de l'entrée dans le digesteur. A la fin du canal d'entrée
48 une fente rectangulaire 49 au fond du canal permet la communication avec l'étage supérieur 21 du compartiment primaire.

- Le canal de sortie 65 a les mêmes caractéristiques que
le canal d'entrée et lui est rigoureusement contigu sur tout son parcours, les produits le parcourt dans le sens de la sortie du digesteur. Il se termine par une lame déversante 66 dont la face supérieure horizontale est exactement au même niveau que le niveau général 68 des liquides dans l'ensemble du digesteur.

Les sections contigues parcourues parallèlement à l'axe longitudinal de la cuve par le canal d'entrée 48 et le canal de sortie 65 sont séparés sur toute leur hauteur par des plaques métalliques 96 d'épaisseur 1 mm environ. Les sections contigues parcourues perpendiculairement à l'axe longitudinal de la cuve par le canal d'entrée 48 et le canal de sortie 65 sont séparés sur toute la hauteur par des cloisons isolantes 98. Les sections contigues parcourues parallèlement à l'axe longitudinal de la cuve par le même canal soit d'entrée 48 soit de sortie 65 sont séparées sur toute la hauteur par des cloisons isolantes 97.

- Dans chaque section de canal d'entrée 48 et de canal de sortie 65 parallèle à l'axe longitudinal du digesteur on a selon les longueurs une ou plusieurs palettes verticales de brassage de largeur légèrement inférieures à la largeur des canaux.

- Le réseau de canalisations permet à partir de la pompe d'introduction 50 et de la pompe de recirculation 67 d'effectuer tous les transferts de produits à l'intérieur de l'appareil.

- Du compartiment secondaire partent des canalisations qui alternativement servent à aspirer les produits devant la cloison 32 et à les refouler derrière ; la même canalisation sert donc tantôt à aspirer tantôt à refouler. Les orifices 52 de ces canalisations sont au fond de la cuve, ils sont tous alignés sur un axe médian dans le sens longitudinal de la cuve, on a un orifice 52 à chaque extrêmité, les orifices intermédiaires sont environ à 2 mètres les uns des autres.

-A chaque extrêmité du compartiment secondaire part une canalisation 61 chacune a un départ qui se situe à 20 cm en-dessous du niveau général 68 des produits puis elle descend verticalement à peu près dans le plan médian près de la paroi, effectue son parcours horizontal dans la dalle, puis elle aboutit au collecteur général d'aspiration 55 par l'intermédiaire d'une vanne.

16

Le collecteur général d'aspiration 55 et le distributeur général de refoulement 57 sont situés côte à côte dans le quart supérieur du compartiment 19, ce sont 2 canalisations à section rectangulaire dont l'axe longitudinal est perpendiculaire à l'axe longitudinal du digesteur, elles sont en charge de 50 cm environ. Toutes les canalisations qui s'y raccordent débouchent à travers leurs parois horizontales inférieures, à chaque débouché de canalisation on a une vanne dont l'élément obturateur est constitué par un disque circulaire dont les actions d'ouverture ou de fermeture sont provoquées par des mouvements rectilignes alternatifs.

- Le collecteur général d'aspiration 55 est relié à l'aspiration de la pompe de recirculation 67, le refoulement de cette pompe débouche dans le distributeur général de refoulement 57. La pompe volumétrique 50 aspire à la base du compartiment primaire et refoule dans l'aspiration de la pompe 67.

- La cloison mobile 32 porte sur chaque face et à la base 2 déflecteurs 106 qui font un angle tel qu'ils ont tendance à ramener les boues du fond dans l'axe central lorsque la cloison 32 avance.

- La cloison 32 est tirée alternativement dans chaque sens par un mécanisme 94 qui est situé dans le local technique 41. La transmission se fait par cable. La vitesse d'avancement est telle que le volume balayé par la cloison 32 pendant un temps donné est égal au débit de la pompe de recirculation 67 pendant ce même temps.

- Les parois verticales 105 du bac d'alimentation 25 du canal de sortie de l'échangeur de chaleur sont situées de part et d'autre de l'échancrure 64 pratiquée à travers la paroi 30 et qui permet aux produits de pénétrer dans le canal, ces 2 parois 105 sont séparées l'une de l'autre par une distance égale à la moitié de la distance qui sépare les 2 parois externes 9, ce bac est alimenté au fond par la canalisation 63.

- Le local technique 41 abrite les mécanismes 94 de traction de la plaque mobile 32 et les mécanismes 95 de déplacement du chariot porteur des divers organes de brassage.

- 17 -

- Le bac à eau 42 reçoit l'ensemble 128 d'épuration compression du biogaz et les gazomètres 129 qui stockent sous une légère pression, avant l'utilisation, le biogaz épuré.

- Par les fenêtres rectangulaires 99, 100 et 101 passent les supports des pales de brassage des 3 étages du compartiment primaire.

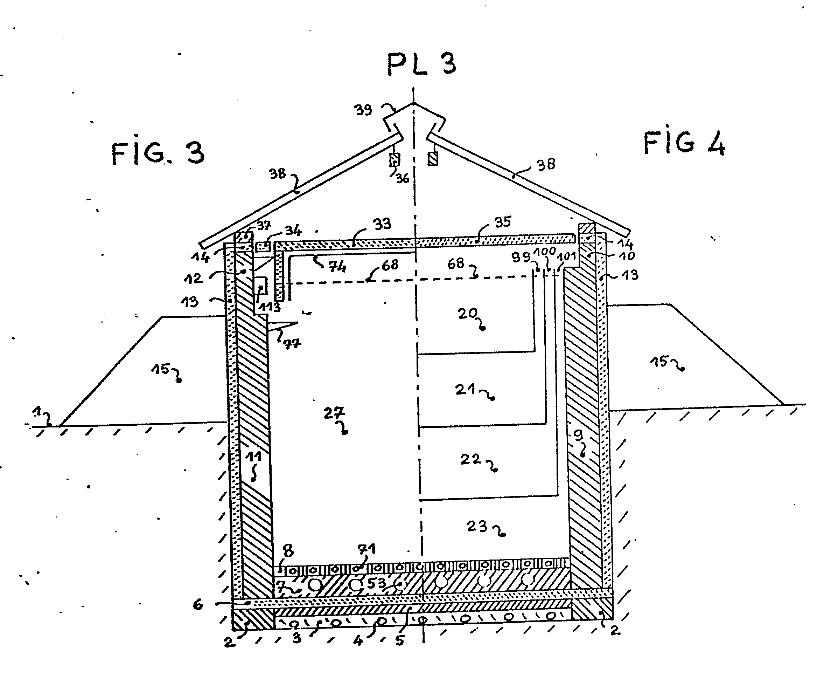

Figure 3 Planche 3

-Sur cette figure on voit la base du digesteur avec la couronne rectangulaire 2 en béton, la zone drainée 3 avec les drains 4, zone surmontée de la dalle béton 5 ; l'ensemble étant recouvert par la couche continue 6 d'isolant incompressible. Au-dessus de cet isolant on a la dalle 7 où sont noyés les tubes tels que 53 où circulent les produits lors de leur transfert, puis encore au-dessus la dalle en béton 8 qui renferme les tubes de chauffage 71.

- La paroi longitudinale 11 a la même épaisseur jusqu'à environ 50 cm en dessous du niveau 68 des produits en cours de fermentation. La paroi 11 est surmontée dans sa partie extérieure par une paroi 12 qui a une épaisseur égale à la moitié de la paroi 11. La paroi 11 porte à sa partie supérieure interne et sur toute la longueur du compartiment secondaire un déflecteur de gaz 77 tandis que la paroi 12 porte tout le long du compartiment secondaire une pièce 113 qui évite le contact des produits avec l'air le long de la paroi 12.

- Les parois 11 et 12 sont recouvertes sur leurs faces externes par une couche continue 13 d'isolant incompressible. La face supérieure de la paroi 12 est recouverte d'une couche continue 14 d'isolant incompressible.

- Au-dessus de la bâche 74 qui sert à recueillir le biogaz on a une couche isolante 33 fixe, sur les côtés on a des panneaux isolants mobiles 34.

- La couverture est assurée par 2 rangées de panneaux 38 qui peuvent basculer de l'extérieur vers l'intérieur, ces panneaux prennent appui à leur partie supérieure sur des supports 36 et à la base sur des supports 37 ; l'ensemble forme un toit à double pente ; la couverture est complétée par une faîtière 39.

Figure 4 Planche 3

- La paroi latérale 9 a la même épaisseur jusqu'à un niveau supérieur de quelques centimètres au niveau général 68 des produits en cours de fermentation. Elle est surmontée dans sa partie externe par une paroi 10 dont l'épaisseur est la moitié de l'épaisseur de la paroi 9. La paroi 9 et la paroi 10 sont recouvertes sur leur face externe d'une couche continue 13 d'isolant incompressible de même la face supérieure de la paroi 10 est recouverte d'une couche continue d'isolant 14. La partie supérieure est isolée par des panneaux 35 amovibles. La hauteur intérieure utile est divisée en 4. De haut en bas on rencontre successivement l'échangeur de chaleur 20 ; le compartiment primaire divisé en 3 étages : l'étage supérieur 21, l'étage moyen 22, l'étage inférieur 23 par deux cloisons horizontales. Chacune d'elle se raccorde sur chaque côté parallèle à l'axe principal du digesteur à une cloison verticale dont la face supérieure dépasse légèrement le niveau général 68 des produits ; de chaque côté les 2 cloisons verticales sont placées de telle façon que de chaque côté on ait 3 espaces libres 99, 100, 101 de 10 cm de large pour permettre le passage des supports des pales de brassage des 3 étages supérieur, moyen et inférieur du compartiment primaire.

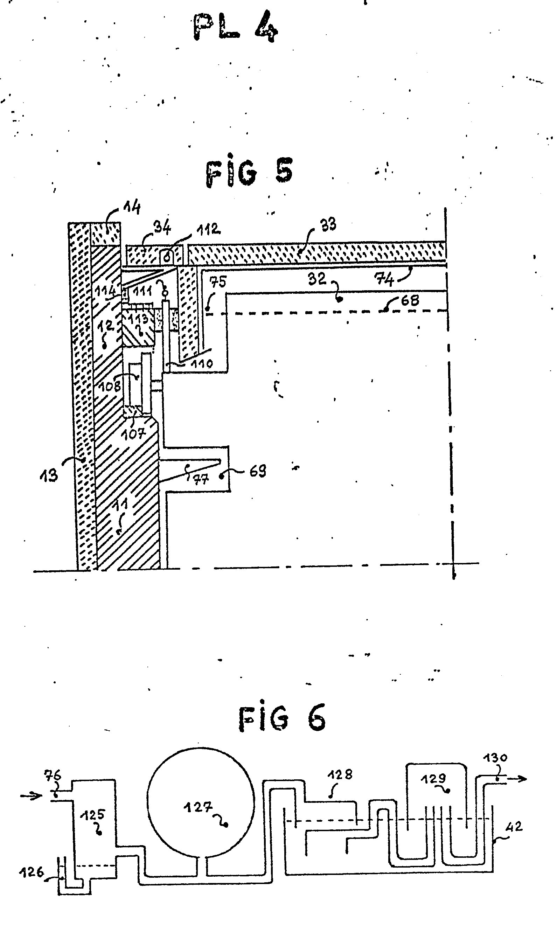

Figure 5 planche 4

- La paroi 11 est surmontée dans sa partie externe par une paroi 12 d'épaisseur égale à la moitié de la paroi 11. L'isolant incompressible 13 et 14 recouvre la face externe de ces parois et la face supérieure de la paroi 12. L'isolation supérieure est assurée au-dessus de la bâche 74 par une couche isolante fixe 33 et sur les côtés par des panneaux amovibles 34 qui permettent la visite des câbles 111 et 112.

- La cloison mobile 32 est suspendue de chaque côté à un galet 108 , chaque galet 108 assure la suspension de la plaque 32 et son guidage transversal et roule sur un chemin de roulement 107. Le chemin de roulement 107 est fixé sur la face supérieure interne de la paroi 11. La face supérieure du chemin de roulement 107 est à un niveau tel que l'axe rectiligne et horizontal qui relie le galet 108 à la paroi mobile 32 puisse passer avec un léger jeu sous le rebord vertical de la bâche 74 de récupération du biogaz.

- La cloison mobile 32 a une surface telle qu'elle coulisse avec un léger jeu contre les parois longitudinales et au fond, sa face supérieure horizontale est située à environ 10 cm au-dessus du niveau général 68 des produits, de chaque côté dans le coin supérieur une échancrure 69 de section rectangulaire la traverse de part en part en permettant le passage du déflecteur de gaz 77 fixé horizontalement le long de la paroi verticale interne de la cuve et le passage des liquides soit dans un sens soit dans l'autre pour équilibrer les niveaux de part et d'autre de la cloison 32.

- La traction de la plaque est assurée de chaque côté par un cable 111 relié à une tige verticale 110 ; elle-même fixée à la paroi mobile. Le point de liaison entre le cable tracteur 111 et la tige verticale 110 se situe à quelques centimètres au-dessus du niveau des produits en fermentation.

- La pièce 113 est fixée tout le long de la face interne de la paroi 12 par des cornières 114.

Figure 6 planche 4

- C'est un schéma du circuit du biogaz. Le biogaz sort du digesteur par la canalisation 76 ; il arrive dans une enceinte 125 à la température ambiante ; l'eau qui se condense est éliminée par le siphon 126. Le biogaz est ensuite stocké dans le gazomètre souple 127. De là, il se rend au système 128 où il est épuré et mis en légère pression puis stocké dans un ou plusieurs gazomètres 129. Le système d'épuration compression 128 est en partie immergé dans la cuve à eau 42, de même le ou les gazomètres 129 sont placés sur cette même cuve à eau. Le biogaz épuré part vers l'utilisation par la canalisation 130.

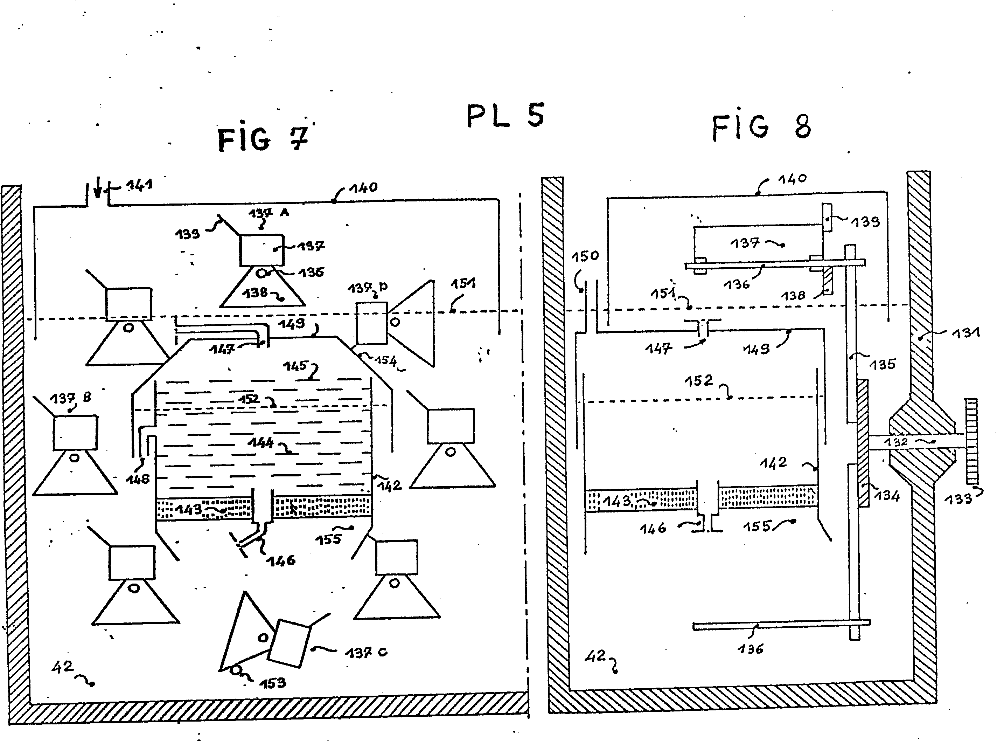

Figure 7 -Planche 5

- Le système d'épuration mise en pression est installé dans la cuve à eau 42. Celle-ci est pleine au trois-quart environ jusqu'au niveau 151. La bâche 140 située sur la cuve à eau reçoit le biogaz par la canalisation 141, l'étanchéité est assurée par le rebord de la bâche qui plonge dans l'eau.

- Les augets sont constitués d'un corps principal 137, ce corps principal est constitué par une boîte parallélipipédique dont l'ouverture est dirigée vers l'axe support 136, à ce corps principal est fixé : à l'angle supérieur une tige ergot 139 et de l'autre côté de l'axe support 136, une plaque triangulaire 138.

- Chaque auget effectue par rapport à l'axe général 132 un mouvement de rotation qui lui permet à la partie haute de sa trajectoire de se remplir de biogaz : position 137 A. Pour cela le plafond de la bâche 140 est à une hauteur suffisante par rapport au niveau 151 de l'eau sous la bâche pour que l'auget effectue une petite partie de sa trajectoire en équilibre stable totalement hors de l'eau. Lors de la descente dans la cuve le système est conçu de telle façon qu'il reste en équilibre stable, c'est-à-dire que le corps principal de l'auget reste tout entier au-dessus de l'axe 136. Le biogaz contenu dans le corps principal est comprimé à mesure que le godet descend : position 137 B. Arrivé au point le plus bas de sa trajectoire une butée 153 détermine le basculement de l'auget et la vidange totale du biogaz emprisonné dans le corps principal : position 137 C. En fin de cycle la came 154 détermine le basculement de l'auget et son maintien dans la position la plus favorable à la vidange de l'eau contenue dans le corps principal à savoir, faces latérales de l'auget parallèles au plan déterminé par la surface de l'eau ; la vidange de l'eau emprisonnée dans le corps principal s'effectue de façon centrifuge dès que le corps principal commence à sortir de l'eau : position 137 D.

- La virolle rectangulaire 142 est placée de façon telle qu'elle recueille à sa base dans la chambre de réception 155 la totalité du biogaz qui s'échappe du corps principal de chaque auget lors de sa vidange au point le plus bas de sa trajectoire.

- Au-dessus de la chambre de réception et sur toute la surface du plafond de celle-ci est placé un diffuseur 143 qui est conçu de telle façon que le gaz après l'avoir traversé de bas en haut ressorte divisé en un maximum de bulles de faible diamètre.

- Au-dessus du diffuseur des chicanes 144 immergées dans l'eau sont disposées de telle façon que les bulles de gaz parcourent un chemin plus long que si elles s'élevaient à peu près verticalement au sein du liquide. Au-dessus du niveau 152 de l'eau à l'intérieur de la virolle les chicanes 145 sont disposées de telle façon qu'étant mouillée par l'eau neuve qui arrive par la canalisation 147 elles permettent un contact maximum entre l'eau qui ruisselle à leur surface et le biogaz environnant.

- Le biogaz épuré est recueilli par une cloche 149 de récupération renversée au-dessus de la partie supérieure de la virolle 142, ses bords latéraux descendent plus bas que le niveau 152 de l'eau ; de la paroi supérieure de la cloche de récupération part une canalisation 150 qui évacue le biogaz épuré vers un gazomètre sous pression.

- L'eau usée qui se trouve à la partie inférieure du volume contenant les chicanes 144 imergées est évacuée par une canalisation 146 qui traverse le diffuseur 143 ; elle est remplacée par de l'eau neuve amenée à la partie supérieure de ce même volume par la canalisation 147.

- Le niveau de l'eau 152 à l'intérieur de la virolle 142 est maintenu identique au-niveau de l'anneau liquide compris entre la virolle 142 et la cloche de récupération 149 par une ouverture 148 pratiquée dans la paroi de la virolle en-dessous du niveau 152 de l'eau.

- Le niveau 152 de l'eau à l'intérieur de la virolle 142 est inférieur au niveau 151 de l'eau sous la bâche 140 de réception du biogaz à traiter.

Figure 8 Planche 5

- L'axe 132 supporté par la paroi 131 de la cuve à eau 42 est commandé de l'extérieur à une de ses extrêmités par une roue dentée motrice 133, il porte à son autre extrêmité une plaque 134 sur laquelle sont fixés des bras supports 135. A la périphérie de chacun de ces bras support 135 est fixé un axe horizontal 136 qui supporte un auget et chaque auget peut tourner autour de son axe support 136 grâce à deux tourillons.

- Chaque auget est composé d'un corps principal 137 qui a la forme d'une boîte parallélipipédique fixée sur l'axe 136 par l'intermédiaire de 2 tourillons qui lui permettent de tourner autour de cet axe ; la face du corps principal tournée vers l'axe est absente de telle sorte que le corps principal est totalement ouvert dans la direction de l'axe. A la face latérale du corps principal située près du bras porteur 135 et symétriquement par rapport à l'axe 136 est fixée une plaque triangulaire 138 dont la masse est telle que l'ensemble de l'auget est en équilibre stable dans la position où le corps principal 137 est tout entier au-dessus de l'axe 136 et la plaque triangulaire 138 toute entière en-dessous de l'axe 136 ; sur cette même face latérale à l'opposé de la plaque triangulaire 138 est fixée une tige ergot 139 qui dans la position d'équilibre stable de l'auget fait un angle de 45° avec l'horizontale.

- Les autres éléments ont déjà été décrits à la figure 7 planche 5.

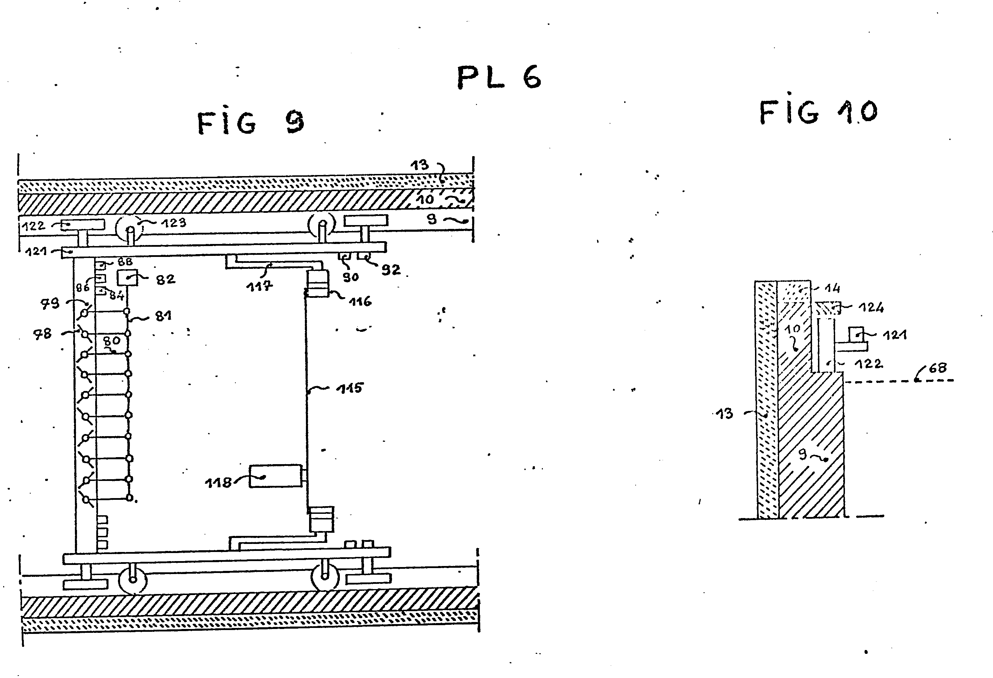

Figure 9 Planche 6

- Cette figure représente la disposition schématique des différents organes de brassage sur le chariot support.

- Le chariot supporte les palettes verticales de brassage 78 du canal d'entrée 48 de l'échangeur de chaleur, les palettes verticales de brassage 79 du canal de sortie 65 de l'échangeur ; ces deux catégories de palettes dont les faces planes (faces de travail) font entre elles des angles de 90° sont toutes reliées par l'intermédiaire de bielettes 80 à une tige de commande d'orientation 81 commandée par un mécanisme 82.

- Le chariot est relié rigidement par l'intermédiaire du support 84 à la pale de brassage de l'étage supérieur du compartiment primaire, par l'intermédiaire du support 86 à la pale de brassage de l'étage moyen, par l'intermédiaire du support 88 à la pale de brassage de l'étage inférieur du compartiment primaire.

- Le chariot est relié rigidement par l'intermédiaire de son support 90 à la pale de brassage du fond du compartiment tertiaire, par l'intermédiaire de son support 92 à la pale de brassage du bac d'alimentation du canal de sortie de l'échangeur de chaleur.

- Le chariot est le support des éléments du brise croûte c'est-à-dire en particulier des bras supports oscillants 117, du support vertical 116 et des organes de travail : plaque rectangulaire 115 et goulotte 118 qui lui est fixée.

- Tous les organes supportés énumérés ci-dessus sont fixés de chaque côté du chariot sur un longeron 121. Il en résulte que les organes de brassage de l'échangeur de chaleur des 3 étages du compartiment primaire ; du compartiment tertiaire ; du bac d'alimentation et le brise croûte du compartiment tertiaire sont tous solidaires mécaniquement.

- A chacune de ses extrêmités chaque longeron 121 prend appui par l'intermédiaire d'un galet 122 à axe horizontal sur le chemin de roulement constitué par la partie supérieure interne de la paroi 9, une pièce est fixée tout le long de la paroi 10 de façon que chaque galet 122 ne puisse décoller de plus de 1 à 2 centimètres de son chemin de roulement.

- A chacune de ses extrêmitée chaque longeron 121 est guidé par un galet 123 à axe vertical, ce galet 123 est situé le plus près possible du galet 122 et son chemin de roulement est constitué par la base de la face interne de la paroi 10.

- Le chariot décrit des mouvements rectilignes alternatifs commandés par un mécanisme moteur situé dans le local technique.

Figure 10 planche 6

- Sur cette figure sont représentés le longeron 121 et un galet porteur 122 à axe horizontal. Le chemin de roulement de ce galet est constitué par la face supérieure de la paroi 9, ce chemin de roulement est à quelques centimètres au-dessus du niveau général 68 des produits. Le galet 122 ne peut décoller de plus de 1 ou 2 cm de son chemin de roulement puisqu'il en est empêché par la pièce 124 qui est fixée à la paroi 10. Les numérotations 13 et 14 représentent les couches d'isolants.

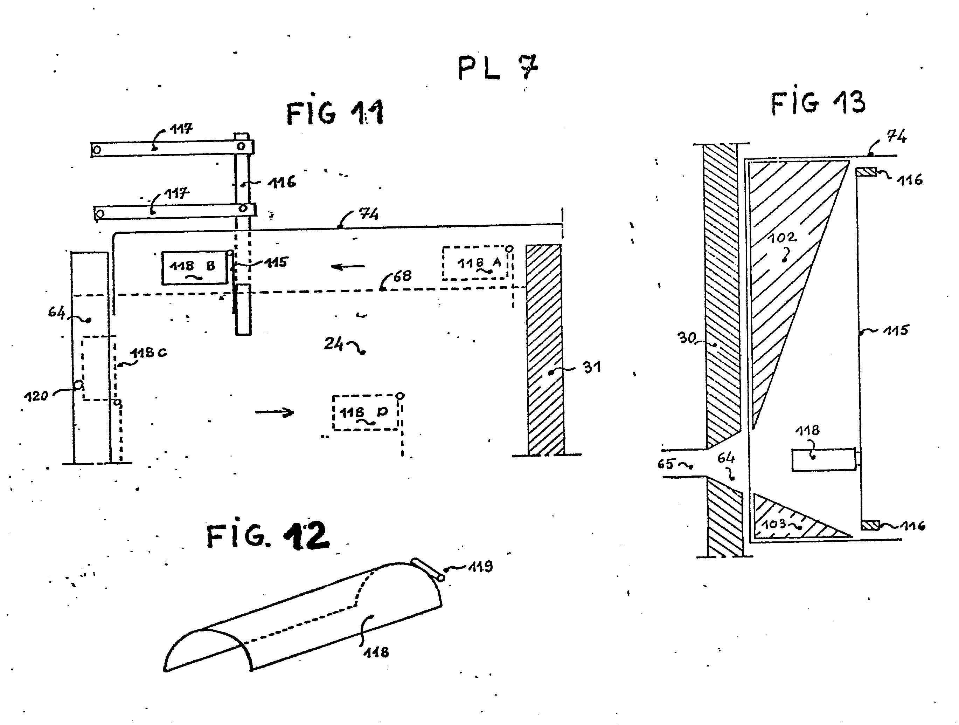

Figure 11 planche 7

- Le brise croûte est constitué principalement d'une plaque métallique rectangulaire 115, reliée à un parallélogramme articulé qui la maintient en permanence verticale, (bras 117).

- Périodiquement la plaque verticale 115, bord inférieur immergé de quelques centimètres pousse d'une extrêmité à l'autre de la partie supérieure du compartiment tertiaire les flottants dans la direction du canal de sortie de l'échangeur de chaleur (paqsage de la position 118 A à la position 118 B). Puis elle s'abaisse tout en continuant le mouvement précédent de façon à ce que la goulotte 118 passe sous le rebord immergé de la bâche 74, le mouvement se continuant jusqu'à ce que la goulotte 118 se redresse verticalement sous l'action de la butée 120 (position 118 C)

- Ensuite la plaque 115 revient à sa position de départ 118 A en se déplaçant d'abord horizontalement dans le liquide de façon que son bord supérieur ne maintienne constamment à quelques centimètres en-dessous du niveau 68 (position 118 D). Puis en fin de parcours un mouvement ascentionnel s'y ajoute de façon à la ramener à son point de départ 118 A.

- Figure 12 planche 7

- On voit que la goulotte 118 est constituée par une demi-virolle circulaire avec un axe horizontal 119 fixé à la génératrice supérieure.

Figure 13 Planche 7

- Sur cette figure qui est une coupe horizontale partielle du compartiment tertiaire au niveau de la plaque 115 du brise-croûte on voit que celle-ci occupe toute la largeur du compartiment à l'exception d'un jeu de quelques centimètres de chaque côté pour éviter le contact avec la bâche de récupération du gaz 74. La plaque rectangulaire verticale 115 a environ 20 cm de haut. Elle est fixée à chacune de ses extrêmités aux supports 116. Elle porte fixée à son arête supérieure par un axe horizontal 119 une goulotte semi-cylindrique 118 ouverte à ses deux extrêmités qui peut pivoter vers le haut d'un angle de 90° à partir de l'horizontale. La goulotte est fixée de telle façon que son plan médian longitudinal coïncide avec le plan médian longitudinal de l'échancrure 64 pratiquée dans la paroi 30 et qui permet la communication avec le canal de sortie de l'échangeur.

- La partie supérieure du compartiment tertiaire comporte 2 déflecteurs 102 et 103 qui sont placés de telle façon que les flottants aient tendance à se concentrer vers le début du canal 65 de sortie de l'échangeur.

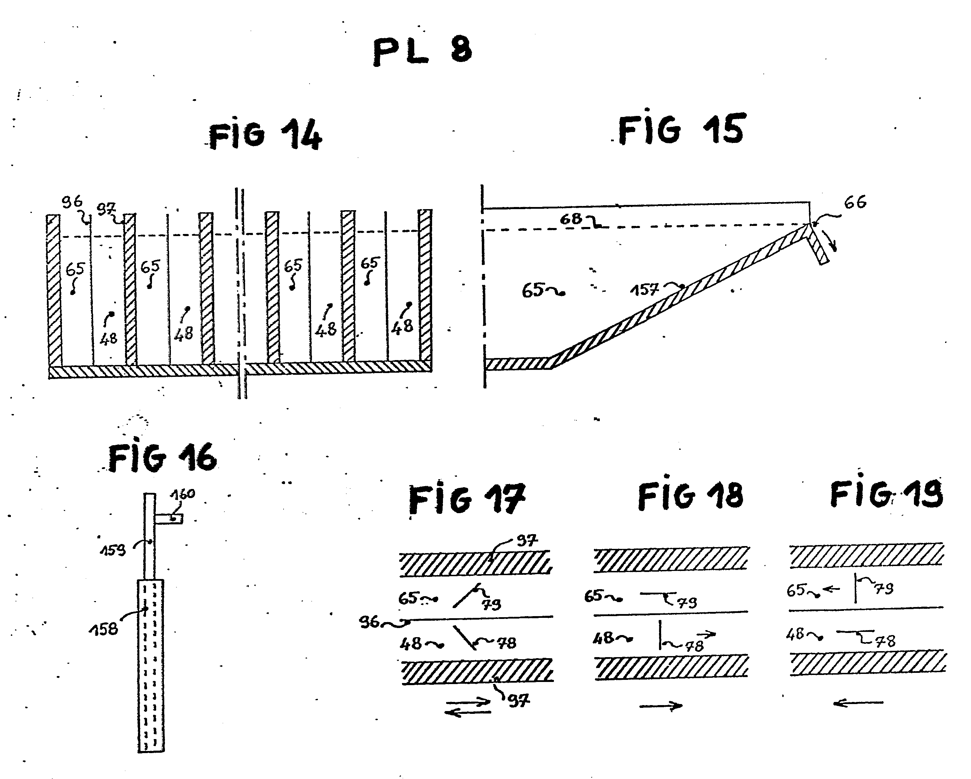

Figures 14 et 15 planche 8

- La figure 14 est une coupe verticale passant par le plan médian de l'échangeur. La figure 15 est une coupe longitudinale à l'extrêmité du canal de sortie 65 de l'échangeur.

- Le fond des canaux, et les parois latérales externes des canaux situés à la périphérie de l'échangeur sont constitués par des parois isolantes 156 et 161.

- Le fond du canal s'achève par une pente continue 157 de 30° par rapport à l'horizontale qui, à la fin, se confond avec la lame déversante 66. Les palettes 79 , poussent mécaniquement les boues sur ce plan incliné et permettent ainsi leurs évacuation.

- Les autres éléments ont été décrits précédemment.

Figure 16 Planche 8

- La figure 16 est une représentation schématique d'une palette de brassage des canaux de l'échangeur de chaleur. Elle est constituée d'un plat 158 fixé sur un axe support 159, le positionnement angulaire de la palette est obtenu par action sur la biêlette 160.

Figures 17, 18 et 19 planche 8

- Les palettes dont la face plane peut présenter des angles variables par rapport au plan médian longitudinal des canaux sont montées sur un axe vertical qui leur communique des mouvements rectilignes d'aller et retour. Les faces planes des palettes peuvent travailler dans 3 positions différentes :

Figure 17

- En position brassage toutes les faces des palettes font un angle de 45° par rapport au plan médian longitudinal des canaux, elles font dans cette position un certain nombre d'aller

0070245

retour.

- Figure 18

      - Périodiquement pour faire avancer les produits dans le canal d'entrée 48 toutes les faces planes des palettes 78 du canal d'entrée 48 font un angle de 90° par rapport au plan médian longitudinal de chaque section de canal : les faces planes des palettes 79 situées dans le canal de sortie 65 font alors un angle de 0° par rapport au même plan ; l'ensemble des palettes 78 et 79 ne fait dans cette position qu'un seul mouvement d'aller.

- Figure 19

      - Immédiatement après le mouvement de retour des palettes sert à faire avancer les produits dans le canal de sortie 65, toutes les faces planes des palettes 79 du canal de sortie 65 font alors un angle de 90° par rapport au plan médian longitudinal du canal et toutes les faces planes des palettes 78 du canal d'entrée 48 font au cours de ce mouvement de retour un angle de 0° par rapport au plan médian longitudinal du canal.

L'ensemble des palettes ne fait dans cette position qu'un seul mouvement de retour.

REVENDICATIONS
=============================

REVENDICATION n° 1

Appareil pour la production en continu de biométhane à partir de substances organiques caractérisé :

- En ce que l'appareil est constitué principalement par une cuve parallélipipédique dont l'axe principal est horizontal

- En ce qu'à l'extrêmité de la cuve la plus proche du compartiment 19 ; et accolée à la face transversale externe 16 se trouve la fosse de régulation 40 surmontée du local technique 41 ; et qu'à l'autre extrêmité est accolée la cuve à eau 42 qui reçoit le système 128 d'épuration mise en pression du biogaz ainsi que les gazomètres 129.

- En ce que cette cuve couverte et isolée est séparée par des cloisons intérieures fixes 29 30 31 en quatre compartiments successivement : un compartiment où sont logés les pompes 50 et 67 et les vannes telles que 56 et 58 ; un compartiment primaire constitué par les volumes 21 , 22 et 23 ; un compartiment tertiaire constitué par le volume 26, un compartiment secondaire constitué par les volumes variables 27 et 28.

- En ce que les produits en cours de digestion passent successivement : dans l'échangeur de chaleur 20 , dans le compartiment primaire (volumes 21, 22, 23), dans le compartiment secondaire (volumes 27 et 28), dans le compartiment tertiaire (volume 26), puis dans l'échangeur 20 où ils cèdent de la chaleur aux produits entrants.

- En ce que toutes les surfaces libres des liquides dans l'ensemble du digesteur sont au même niveau général 68.

- En ce que le compartiment secondaire où s'effectue principalement la digestion est séparé en 2 par une cloison mobile 32 qui se déplace alternativement d'un bout à l'autre de ce compartiment pendant que la pompe de recirculation 67 aspire les produits devant la cloison mobile 32 pour les refouler derrière en même temps que sont injectés dans la veine des produits en recirculation, les produits en provenance du compartiment primaire à l'aide de la pompe d'introduction 50.

- En ce que le compartiment tertiaire 26 est équipé d'un bac 25 d'alimentation du canal de sortie de l'échangeur de chaleur et à sa partie supérieure 24 d'un brise croûte constitué principalement d'une plaque métallique rectangulaire 115.

- En ce qu'un réseau de canalisations permet à partir de la pompe d'introduction 50 et de la pompe de recirculation 67 d'effectuer tous les transferts de produits à l'intérieur de l'appareil.

- En ce que les organes de brassage de l'échangeur de chaleur 20 ; des 3 étages 21, 22 et 23 du compartiment primaire ; du compartiment tertiaire 26 ; du bac d'alimentation 25 et le brise croûte du compartiment tertiaire sont tous solidaires mécaniquement.

- En ce que le biogaz est recueilli par une bâche souple 74 à la surface des compartiments secondaire (volumes 27 et 28) et tertiaire (volume 26).

- En ce que le biométhane avant d'être prêt à être utilisé passe dans un dispositif qui réalise simultanément son épuration et sa mise en pression.

REVENDICATION n° 2

- Appareil selon la revendication 1 comprenant une cuve parallélipipédique dont l'axe principal est horizontal caractérisée :

- En ce que les parois latérales du digesteur prennent appui sur le sol par l'intermédiaire d'une couronne rectangulaire en béton 2. La section verticale de cette couronne est rectangulaire ; cette section a comme dimensions horizontales la largeur des parois latérales à la base augmentée de l'épaisseur de l'isolant. Le volume situé à l'intérieur de cette couronne est occupé à la base par une zone drainante 3 et à la partie supérieure par une dalle en béton 5.

- En ce que toute la surface de la couronne 2 et de la dalle 5 est recouverte par une couche continue d'isolant incompressible 6.

- En ce que les parois latérales externes en béton (parois transversales 16 et parois longitudinales 9 et 11) prennent appui à leur base sur la périphérie de la couche iso- lante 6 et que le reste de la couche isolante 6 est surmonté par une dalle 7 en béton dans laquelle sont noyés tous les tubes de transfert de produits.

- En ce que la dalle 7 en béton est surmontée par une dalle 8 en béton dans laquelle sont noyés les tubes de chauffage 71 des produits en cours de digestion sauf sur la surface occupée par le compartiment 19.

- En ce que les parois longitudinales externes situées en face du compartiment 19, du compartiment primaire (volumes 21, 22, 23), du compartiment tertiaire 26 sont constituées jusqu'à une hauteur supérieure de quelques cm à celle du niveau général 68 des produits par une paroi 9 en béton ; au-dessus le reste est constitué par une paroi 10 en béton située à l'extérieur et d'épaisseur égale à la moitié de celle de la paroi 9; le décrochement dont la largeur est égale à la moitié de l'épaisseur de la paroi 9 est situé à l'intérieur.

- En ce que les parois longitudinales externes situées en face du compartiment secondaire sont constituées jusqu'à une hauteur inférieure de 50 cm environ au niveau général 68 des produits par une paroi en béton 11 ; au-dessus, le reste est constitué par une paroi 12 en béton situé à l'extérieur et d'épaisseur égale à la moitié de la paroi 11 ; le décrochement dont la largeur est égale à la moitié de l'épaisseur de la paroi 11 est situé vers l'intérieur.

- En ce que toute la surface externe des parois latérales en béton de la cuve est recouverte de couches continues 13 et 17, d'isolant incompressible.

- En ce que toute la surface des faces supérieures des parois latérales externes en béton de la cuve est recouverte de couches 14 et 18 d'isolant incompressible.

– 30 –

- En ce que la couverture est assurée par 2 rangées de panneaux mobiles 38 formant un toit à double pente, tous ces panneaux 38 basculent de l'extérieur du digesteur vers l'intérieur du digesteur.

- En ce que à la partie supérieure ; la surface située au-dessus du compartiment 19 , et au-dessus de l'échangeur 20 est intégralement recouverte par des panneaux isolants amovibles 35 qui laissent un volume libre de 0,50 m environ au-dessus du niveau général des produits, le reste de la surface, c'est-à-dire celle située au-dessus du compartiment tertiaire 26 et du compartiment secondaire 27, 28 est intégralement recouverte d'une couche d'isolants de la façon suivante ; la surface située au-dessus de la bâche de récupération 74 du biogaz est recouverte par des panneaux isolants fixes 33, le volume laissé libre au-dessus du niveau général des produits est de 0,30 m environ, le reste de la surface, le long des parois latérales est recouvert par des panneaux isolants amovibles 34.

- En ce que trois cloisons fixes verticales · 29, 30 et 31 cloisonnent sur toute la section et perpendiculairement à l'axe longitudinal de la cuve l'intérieur de la cuve et déterminent 4 compartiments successivement et dans l'ordre ; un compartiment 19 où sont logées principalement les vannes , puis un compartiment qui est occupé à l'étage supérieur par l'échangeur de chaleur 20 et en-dessous par le compartiment appelé compartiment primaire (volume 21, 22 et 23) puis un compartiment appelé compartiment tertiaire 26 et enfin un compartiment appelé compartiment secondaire (volumes variables 27 et 28).

REVENDICATION n° 3

- Appareil selon la revendication 1 comprenant un compartiment primaire (volumes 21, 22, 23) caractérisé :

- En ce que ce compartiment situé sous l'échangeur de chaleur 20 a une hauteur totale égale au trois-quart de la hauteur déterminée par le niveau constant 68 du liquide.

- En ce qu'il est divisé en 3 étages de même hauteur intérieure par 2 cloisons horizontales qui déterminent donc : un étage supérieur 21, un étage moyen 22 et un étage inférieur 23 ; et en ce que chacune des 2 cloisons horizontales se raccorde sur chaque côté parallèle à l'axe principal du digesteur à une cloison verticale dont la face supérieure dépasse légèrement le niveau général 68 des produits dans le digesteur, de chaque côté les 2 cloisons verticales sont placées de telle façon que de chaque côté on ait 3 espaces libres : 99, 100, 101, de 10 cm de large environ.

- En ce que chacun des 3 étages 21, 22 et 23 est brassé par des pales 83, 85, 87 animées d'un mouvement rectiligne alternatif, les bras de fixation des pales qui les soutiennent et les entrainent mécaniquement passent dans les espaces libres 99, 100 et 101 définis à la fin de l'alinéa précédent.

- En ce que les produits passent de l'étage supérieur 21 à l'étage moyen 22 puis à l'étage inférieur 23 par des ouvertures situées de telle façon que le parcours des produits soit maximum.

- En ce que les produits sont prélevés à l'étage inférieur 23 de telle façon qu'ils aient parcouru le chemin maximum par une canalisation reliée à une pompe volumétrique 50 qui les envoie à l'aide de 2 canalisations équipées chacune d'une vanne.

- Soit à l'entrée de l'étage supérieur du compartiment primaire 21 pour ensemencer les produits frais.

- Soit dans la canalisation reliée à l'aspiration de la pompe de recirculation 67 (avec la canalisation 51)

- En ce que son volume total est tel que les produits y séjournent environ 36 heures.

REVENDICATION n° 4

- Appareil selon la revendication 1 comprenant un échangeur de chaleur 20 caractérisé :

- En ce qu'il occupe un volume situé au-dessus du compartiment primaire, ce volume a pour surface de base la surface du compartiment primaire moins une bande de 30 cm de large contre chacune des 2 faces longitudinales et pour hauteur le quart de la hauteur déterminé par le niveau constant 68 du liquide.

- En ce qu'il comprend un canal d'entrée 48 des produits ; de section rectangulaire constante ayant pour largeur 10 cm environ et pour hauteur la hauteur de l'échangeur;à son début ce canal traverse le compartiment 19, il pénêtre ensuite dans la zone occupée par l'échangeur proprement dit qu'il parcourt en faisant des allers et retours successifs de façon à parcourir la distance maximum, les produits le parcourt dans le sens de l'entrée dans le digesteur.

- En ce qu'il comprend un canal de sortie 65, qui a les mêmes caractéristiques que le canal d'entrée et lui est rigoureusement contigu sur tout son parcours, les produits le parcourt dans le sens de la sortie du digesteur.

- En ce que dans les sections contigues parcourues parallèlement à l'axe longitudinal de la cuve le canal d'entrée 48 et le canal de sortie 65 sont séparés sur toute leur hauteur par des plaques métalliques 96 d'épaisseur 1 mm environ

- En ce que dans les sections contigues parcourues perpendiculairement à l'axe longitudinal de la cuve le canal d'entrée 48 et le canal de sortie 65 sont séparés sur toute la hauteur par des cloisons isolantes 98.

- En ce que dans les sections contigues parcourues parallèlement à l'axe longitudinal de la cuve par le même canal soit d'entrée 48 soit de sortie 65 les 2 sections sont séparées sur toute la hauteur par des cloisons isolantes 97.

- En ce que le fond des canaux, et les parois latérales externes des canaux situés à la périphérie de l'échangeur sont constitués par des parois isolantes 156 et 161.

- En ce qu'au début du canal d'entrée 48 on a une lame déversante 47 dont la face supérieure horizontale est exactement au même niveau que le niveau général 68 des liquides dans l'ensemble du digesteur.

- En ce qu'à la fin du canal d'entrée 48 une fente rectangulaire 49 au fond du canal permet la communication avec l'étage supérieur 21 du compartiment primaire.

- En ce que le canal de sortie 65 se termine par une lame déversante 66 dont la face supérieure horizontale est exactement au même niveau que le niveau général 68 des liquides dans l'ensemble du digesteur, et que le fond du canal s'achève par une pente continue 157 de 30° par rapport à l'horizontale qui, à la fin du canal se confond avec la lame déversante 66.

- En ce que dans chaque section de canal d'entrée 48 et de canal de sortie 65 parallèle à l'axe longitudinal du digesteur on a selon la longueur des sections une ou plusieurs palettes verticales de largeur légèrement inférieure à la largeur du canal, ces palettes dont la face plane 158 peut présenter des angles variables par rapport au plan médian longitudinal des canaux sont montées sur un axe vertical 159 qui leur communique des mouvements rectilignes d'aller et retour, les faces planes 159 des palettes peuvent travailler dans 3 positions différentes :

-En position brassage toutes les faces 158 des palettes font un angle de 45° par rapport au plan médian longitudinal des canaux, elles font dans cette position un certain nombre d'aller et retour.

- Périodiquement pour faire avancer les produits dans le canal d'entrée 48 toutes les faces planes 158 des palettes du canal d'entrée 48 font un angle de 90° par rapport au plan médian longitudinal de chaque section de canal : les faces planes 158 des palettes situées dans le canal de sortie 65 font alors un angle de 0° par rapport au même plan ; l'ensemble des palettes ne fait dans cette position qu'un seul mouvement d'aller.

-Immédiatement après le mouvement de retour des palettes sert à faire avancer les produits dans le canal de sortie 65 ; toutes les faces planes 158 des palettes du canal de sortie 65 font alors un angle de 90° par rapport au plan médian longitudinal du canal et toutes les faces planes 158 des palettes du canal d'entrée 48 font au cours de ce mouvement de retour un angle de 0° par rapport au plan médian longitudinal du canal. L'ensemble des palettes ne fait dans cette position qu'un seul mouvement de retour.

REVENDICATION n°5

– Appareil selon la revendication 1 comprenant un compartiment secondaire caractérisé :

– En ce que ce compartiment secondaire est divisé en 2 sous-compartiments 27 et 28 de volume variable par une cloison mobile 32 qui peut se déplacer d'un mouvement rectiligne alternativement d'un bout à l'autre de ce compartiment jusqu'à 20 cm environ de chaque extrêmité. Le déplacement se fait parallélement à l'axe longitudinal du digesteur.

– En ce que la cloison mobile 32 a une surface telle qu'elle coulisse avec un léger jeu contre les parois longitudinales et au fond, que sa face supérieure horizontale est située à environ 10 cm au-dessus du niveau général 68 des produits, que de chaque côté dans le coin supérieur une échancrure 69 de section rectangulaire la traverse de part en part en permettant le passage du déflecteur de gaz 77 fixé horizontalement le long de la paroi verticale interne de la cuve et le passage des liquides soit dans un sens soit dans l'autre pour équilibrer les niveaux de part et d'autre de la cloison 32.

– En ce que la cloison mobile 32 porte sur chaque face et à la base 2 déflecteurs 106 qui font un angle tel qu'ils ont tendance à ramener les boues du fond dans l'axe central lorsque la cloison 32 avance.

– En ce que la cloison mobile 32 est suspendue de chaque côté à un galet 108. La cloison 32 est tirée alternativement dans chaque sens par un mécanisme 94 qui est situé dans le local technique 41, la transmission se fait par cable. La vitesse d'avancement est telle que le volume balayé par la cloison 32 pendant un temps donné est égal au débit de la pompe de recirculation 67 pendant ce même temps.

– En ce que chaque galet 108 qui assure la suspension de la plaque 32 et son guidage transversal roule sur un chemin de roulement 107. Ce chemin de roulement 107 est fixé sur la face supérieure interne de la paroi 11. La face supérieure du chemin de roulement 107 est à un niveau tel que l'axe rectiligne et horizontal qui relie le galet 108 à la paroi mobile 32 puisse passer avec un léger jeu sous le rebord vertical de la bâche 74 de récupération du biogaz.

- En ce que la traction de la plaque est assurée de chaque côté par un cable 111 relié à une tige verticale 110 elle-même fixée à la paroi mobile. Le point de liaison entre le cable tracteur 111 et la tige verticale 110 se situe à quelques centimètres au-dessus du niveau des produits en fermentation.

REVENDICATION n° 6

- Appareil selon la revendication 1 comprenant un compartiment tertiaire 26 caractérisé :

- En ce que son volume est tel que les produits y séjournent 1 jour ou 2 si le but recherché est la production de biométhane et qu'en épuration son volume est fonction de la diminution de $DBO_5$ recherché.

- En ce qu'il communique avec le compartiment secondaire par une ouverture 70 de 20 cm de diamètre environ qui traverse la paroi 31 qui sépare les 2 compartiments en son centre.

- En ce qu'à sa partie supérieure se trouve un bac d'alimentation 25 qui alimente le canal 65 de sortie de l'échangeur à travers une échancrure 64 dans la paroi 30 de séparation.

- En ce que le fond 104 du bac d'alimentation est sensiblement à la même hauteur que le fond du canal de sortie 65 de l'échangeur de chaleur, et qu'il est constitué de 2 panneaux légèrement inclinés l'un vers l'autre de façon à faire à leur ligne de jonction une petite rigole elle-même inclinée vers l'entrée du canal de sortie de l'échangeur de chaleur.

- En ce que les parois latérales verticales 105 de ce bac ont des dimensions telles que leurs faces supérieures horizontales soient à environ la moitié de la hauteur du canal de sortie de l'échangeur de chaleur.

    - En ce que les parois latérales verticales 105 sont à une distance l'une de l'autre égale à la moitié environ de la largeur intérieure de la cuve.

    - En ce que le bac 25 est alimenté par une conduite 63 qui débouche au fond du bac, et en ce qu'il est également alimenté par surverse au-dessus de la face supérieure horizontale des parois latérales 105, les dites faces supérieures se trouvant en-dessous du niveau général 68 des produits.

    - En ce que le fond du compartiment lui-même et le bac d'alimentation du canal de sortie de l'échangeur sont brassés respectivement par les pales 89 et 91 animées d'un mouvement rectiligne alternatif.

    - En ce que le brise croûte comporte une plaque rectangulaire verticale 115 d'environ 20 cm de haut et de longueur telle qu'elle laisse de chaque côté un jeu de quelques centimètres par rapport au rebord vertical de la bâche 74 de récupération du biogaz.

    - En ce que la plaque 115 est reliée à un parallélogramme articulé qui la maintient en permanence verticale.

    - En ce que la plaque verticale 115 porte fixée à son arête supérieure par un axe horizontal 119 une goulotte semi-cylindrique 118 ouverte à ses deux extrémités qui peut pivoter vers le haut d'un angle de 90° à partir de l'horizontale. La goulotte est fixée de telle façon que son plan médian longitudinal coïncide avec le plan médian longitudinal de l'échancrure 64 pratiquée dans la paroi 30 et qui permet la communication avec le canal de sortie de l'échangeur.

    - En ce que la partie supérieure du compartiment tertiaire comporte 2 déflecteurs 102 et 103 qui sont placés de telle façon que les flottants aient tendance à se concentrer vers le début du canal de sortie de l'échangeur.

    - En ce que périodiquement la plaque verticale 115, bord inférieur immergé de quelques centimètres pousse d'une extrémité à l'autre de la partie supérieure du compartiment tertiaire les flottants dans la direction du canal de sortie de l'échangeur de chaleur. Puis en ce qu'elle s'abaisse tout en continuant le mouvement précédent de façon à ce que la goulotte 118 passe sous le rebord immergé de la bâche 74, le mouvement se continuant jusqu'à ce que la goulotte 118 se redresse verticalement sous l'action de la butée 120.

- En ce que la plaque 115 revient à sa position de départ en se déplaçant d'abord horizontalement dans le liquide de façon que son bord supérieur se maintienne constamment à quelques centimètres en dessous du niveau 68 des produits puis en fin de parcours en ajoutant à ce mouvement de déplacement un mouvement ascentionnel qui la ramène à son point de départ.


## REVENDICATION n° 7

- Appareil selon la revendication 1 comportant un système de canalisations caractérisées :

- En ce que du fond du compartiment secondaire partent des canalisations 53 qui alternativement servent à aspirer les produits devant la cloison 32 et à les refouler derrière ; la même canalisation sert donc tantôt à aspirer tantôt à refouler. Les orifices 52 de ces canalisations 53 sont au fond de la cuve, ils sont tous alignés sur un axe médian dans le sens longitudinal de la cuve, on a un orifice 52 à chaque extrêmité, les orifices intermédiaires sont environ à 2 mètres les uns des autres.

- Ces canalisations 53 après un parcours horizontal dans la dalle 7 située immédiatement au-dessus de l'isolant 6 arrivent toutes à la base du compartiment 19 ; là, elles se divisent chacune en 2 tronçons ascendants 54 et 59, l'un 54 par l'intermédiaire de vannes 56 est relié au collecteur général d'aspiration 55, l'autre 59 par l'intermédiaire de vannes 58 est relié au distributeur général de refoulement 57.

- En ce qu'à chaque extrêmité du compartiment secondaire part une canalisation 61 ; chacune a un départ qui se situe à 20 cm en-dessous du niveau général 68 des produits puis elle descend verticalement à peu près dans le plan médian près de la paroi, effectue son parcours horizontal dans la dalle 7 et remonte ensuite verticalement dans le compartiment 19 où par l'intermédiaire de vannes elles sont reliées au collecteur général d'aspiration 55.

- En ce que du fond du compartiment tertiaire 26 part une canalisation 62 qui a exactement les mêmes caractéristiques de parcours et de relations que les canalisations 53 qui partent du fond du compartiment secondaire.

- En ce qu'une canalisation 63 alimente par le fond le bac d'alimentation 25. Cette canalisation est reliée au distributeur général de refoulement 57 par l'intermédiaire d'une vanne.

- En ce que toutes les canalisations reliées soit au compartiment secondaire, soit au compartiment tertiaire et dans lesquelles il faut exercer une aspiration sont reliées au collecteur général d'aspiration 55 et qu'à chaque fois cette liaison est réalisée par l'intermédiaire d'une vanne.

- En ce que toutes les canalisations reliées soit au compartiment secondaire, soit au compartiment tertiaire, soit au bac d'alimentation 25 du canal de sortie de l'échangeur et dans lesquelles il faut refouler des produits sont reliés au distributeur général de refoulement 57 et que pour chaque canalisation concernée cette liaison est réalisée par l'intermédiaire d'une vanne.

- En ce que le collecteur général d'aspiration 55 et le distributeur général de refoulement 57 sont situés côte à côte dans le quart supérieur du compartiment 19 ; que ce sont 2 canalisations à section rectangulaire dont l'axe longitudinale est perpendiculaire à l'axe longitudinale du digesteur ; qu'elles sont en charge de 50 cm environ; que toutes les canalisations qui s'y raccordent débouchent à travers leurs parois horizontales inférieures, qu'à chaque débouché de canalisation on a une vanne dont l'élément obturateur est constitué par un disque circulaire dont les actions d'ouverture ou de fermeture sont provoquées par des mouvements rectilignes alternatifs.

- En ce que le collecteur général d'aspiration 55 est relié à l'aspiration de la pompe de recirculation 67 ; que le refoulement de cette pompe débouche dans le distributeur général de refoulement 57 et que le refoulement de la pompe volumétrique 50 qui transporte les produits en provenance du compartiment primaire est également relié à l'aspiration de cette pompe 67.

REVENDICATION n° 8

-Appareil selon la revendication 1 comprenant un chariot support des organes de brassage caractérisé :

- En ce que ce chariot supporte les palettes verticales de brassage 78 du canal d'entrée 48 de l'échangeur de chaleur, les palettes verticales de brassage 79 du canal de sortie 65 de l'échangeur ; que ces deux catégories de palettes dont les faces planes (faces de travail) font entre elles des angles de 90° sont toutes reliées par l'intermédiaire de bielettes 80 à une tige de commande d'orientation 81 commandée par un mécanisme 82.

- En ce que ce chariot est relié rigidement par l'intermédiaire du support 84 à la pale 83 de brassage de l'étage supérieur du compartiment primaire, par l'intermédiaire du support 86 à la pale 85 de brassage de l'étage moyen, par l'intermédiaire du support 88 à la pale 87 de brassage de l'étage inférieur du compartiment primaire.

- En ce que ce chariot est relié rigidement par l'intermédiaire de son support 90 à la pale de brassage 89 du fond du compartiment tertiaire, par l'intermédiaire de son support 92 à la pale de brassage 91 du bac d'alimentation du canal de sortie de l'échangeur de chaleur.

- En ce que ce chariot est le support des éléments du brise croûte c'est-à-dire en particulier des bras supports oscillants 117. du support vertical 116 et des organes de travail : plaque rectangulaire 115 et goulotte 118 qui lui est fixée.

- En ce que tous les organes supportés et commandés sont fixés de chaque côté du chariot sur un longeron 121.

- En ce qu'à chacune de ses extrêmités chaque longeron 121 prend appui par l'intermédiaire d'un galet 122 à axe horizontal sur le chemin de roulement constitué par la partie supérieure interne de la paroi 9, qu'une pièce 124 est fixée tout le long de la paroi 10 de façon que chaque galet 122 ne puisse décoller de plus de 1 à 2 centimètres de son chemin de roulement.

- En ce qu'à chacune de ses extrêmités chaque longeron 121 est guidé par un galet 123 à axe vertical, que ce galet 123 est situé le plus près possible du galet 122 et que son chemin de roulement est constitué par la base de la face interne de la paroi 10.

- En ce que ce chariot décrit des mouvements rectilignes alternatifs commandés par un mécanisme moteur 95 situé dans le local technique 41.

REVENDICATION n° 9

- Appareil selon la revendication 1 comprenant un système de récupération du biogaz caractérisé :

- En ce que la récupération du biogaz s'effectue au-dessus du compartiment secondaire et du compartiment tertiaire

- En ce que la récupération se fait par une bâche . souple 74 dont les bords latéraux plongent dans les produits en digestion assurant ainsi l'étanchéité par un joint hydraulique 75

-En ce que le départ du gaz s'effectue par une canalisation 76 dont le départ se situe à travers la face supérieure de la bâche.

REVENDICATION n° 10

- Appareil selon la revendication 1 comprenant un système d'épuration et de mise en pression du biogaz caractérisé:

- En ce que le système comporte une cuve 42 remplie d'eau au trois-quart de sa hauteur.

- En ce que sur cette cuve à eau 42 est située une bâche 140 de réception du biogaz à traiter, que les bords latéraux inférieurs de cette bâche plongent dans l'eau afin d'assurer un joint hydraulique et que cette bâche reçoit par une canalisation 141 le biogaz à traiter qui a été stocké provisoirement dans un réservoir souple 127 après avoir été recueilli par la bâche souple 74 à la surface des compartiments secondaire et tertiaire du digesteur.

- En ce qu'un axe 132 supporté par la paroi 131 de la cuve à eau 42 et commandé de l'extérieur à une de ses extrêmités par une roue dentée motrice 133 porte à son autre extrêmité une plaque 134 sur laquelle sont fixés des bras supports 135, qu'à la périphérie de chacun de ces bras support 135 est fixé un axe horizontal 136 qui supporte un auget et que chaque auget peut tourner autour de son axe support 136 grâce à deux tourillons.

- En ce que chaque auget est composé d'un corps principal 137 qui a la forme d'une boîte parallélipipédique fixée sur l'axe 136 par l'intermédiaire de 2 tourillons qui lui permettent de tourner autour de cet axe ; que la face du corps principal tournée vers l'axe est absente de telle sorte que le corps principal est totalement ouvert dans la direction de l'axe ; qu'à la face latérale du corps principal située près du bras porteur 135 et symétriquement par rapport à l'axe 136 est fixée une plaque triangulaire 138 dont la masse est telle que l'ensemble de l'auget soit en équilibre stable dans la position où le corps principal 137 est tout entier au-dessus de l'axe 136 et la plaque triangulaire 138 toute entière en-dessous de l'axe 136 et que sur cette même face latérale à l'opposé de la plaque triangulaire 138 est fixée une tige ergot 139 qui dans la position d'équilibre stable de l'auget fait un angle de 45° avec l'horizontale.

- En ce que chaque auget effectue par rapport à l'axe général 132 un mouvement de rotation qui lui permet à la partie haute de sa trajectoire de se remplir de biogaz ; pour cela la plafond de la bâche 140 est à une hauteur suffisante par rapport au niveau 151 de l'eau sous la bâche pour que l'auget effectue une petite partie de sa trajectoire en équilibre stable totalement hors de l'eau ; que lors de la descente dans la cuve le système est conçu de telle façon qu'il reste en équilibre stable, c'est-à-dire que le corps principal de l'auget est tout entier au-dessus de l'axe 136 ; qu'arrivée au point le plus bas de sa trajectoire une butée 153 détermine le basculement de l'auget et la vidange totale du biogaz emprisonné dans le corps principal ; et qu'en fin de cycle la came 154 détermine le basculement de l'auget et son maintien dans la position la plus favorable à la vidange de l'eau contenue dans le corps principal à savoir : faces latérales parallèles au plan déterminé par la surface de l'eau de façon que la vidange de l'eau emprisonnée dans le corps principal s'effectue de façon centrifuge dès que le corps principal commence à sortir de l'eau.

- En ce qu'une virolle rectangulaire 142 est placée de façon telle qu'elle recueille à sa base dans la chambre de réception 155 la totalité du biogaz qui s'échappe du corps principal de chaque auget lors de sa vidange au point le plus bas de sa trajectoire ; qu'au-dessus de la chambre de réception et sur toute la surface du plafond de celle-ci est placé un diffuseur 143 qui est conçu de telle façon que le gaz après l'avoir traversé de bas en haut ressorte divisé en un maximum de bulles de faible diamètre, qu'au-dessus du diffuseur des chicanes 144 immergées dans l'eau sont disposées de telle façon que les bulles de gaz parcourent un chemin plus long que si elles s'élevaient à peu près veticalement au sein du liquide ; qu'au-dessus du niveau 152 de l'eau à l'intérieur de la virolle des chicanes 145 sont disposées de telle façon qu'étant mouillée par l'eau neuve qui arrive par la canalisation 147 elles permettent un contact maximum entre l'eau qui ruisselle à leur surface et le biogaz environnant, que le biogaz épuré est recueilli par une cloche 149 de récupération renversée au-dessus de la partie supérieure de la virolle 142 et que de la paroi supérieure de la cloche de récupération part une canalisation 150 qui évacue le biogaz épuré vers un gazomètre sous pression.

- 43 -

- En ce que l'eau qui se trouve à la partie inférieure du volume contenant les chicanes 144 imergées est évacuée par une canalisation 146 qui traverse le diffuseur 143 et qu'elle est remplacée par de l'eau amenée à la partie supérieure de ce même volume par la canalisation 147.

- En ce que le niveau de l'eau 152 à l'intérieur de la virolle 142 est maintenu identique au niveau de l'anneau liquide compris entre la virolle 142 et la cloche de récupération 149 par une ouverture 148 pratiquée dans la paroi de la virolle en-dessous du niveau 152 de l'eau.

- En ce que le niveau 152 de l'eau à l'intérieur de la virolle 142 est inférieur au niveau 151 de l'eau sous la bâche 140 de réception du biogaz à traiter.

PL 1    FIG. 1

0070245

PL2 FIG. 2

FIG. 3      FIG 4

PL 3

PL 4

## FIG 5

## FIG 6

PL 5

FIG 7

FIG 8

FIG 9

FIG 10

FIG 11

117

117

116

74

118 B 115 118 A

68

64

118 C

120 24

118 D

31

FIG. 12

119

118

FIG 13

74

116

102

30 115

118

65 64

103 116

PL 8

FIG 14

FIG 15

FIG 16

FIG 17

FIG 18

FIG 19

0070245

## RAPPORT DE RECHERCHE EUROPEENNE

Office européen
des brevets

Numéro de la demande

EP 82 42 0084

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| A | US-A-3 954 619 (LUCIUS JOHN FRY) | | C 12 M 1/00 |
| | ----- | | |

**DOMAINES TECHNIQUES RECHERCHES (Int. Cl. 3)**

C 12 M

Le présent rapport de recherche a été établi pour toutes les revendications

| Lieu de la recherche | Date d'achèvement de la recherche | Examinateur |
|---|---|---|
| LA HAYE | 07-10-1982 | COUCKE A.O.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82